# EUROPÄISCHE PATENTANMELDUNG

(11) **EP 1 319 394 A1**
(43) Veröffentlichungstag der Anmeldung: **18.06.2003**
(21) Anmeldenummer: 02026632.6
(22) Anmeldetag: 29.11.2002
(51) Int. Cl.: A61K 7/32, A61L 9/01

(54) **Zubereitungen mit desodorierender Wirkung, enthaltend das Zinksalz der Ricinolsäure und mindestens eine aminofunktionelle Aminosäure**

(30) Priorität: 12.12.2001 DE 10160933
(71) Anmelder: Goldschmidt AG, 45127 Essen (DE)
(72) Erfinder: Lersch, Peter, Dr., 46537 Dinslaken (DE); Müller, Felix, Dr., 42555 Velbert (DE); Peggau, Jörg, 45357 Essen (DE); Ulrich, Patrick, 45143 Essen (DE)

(57) **Zusammenfassung**

Gegenstand der Erfindung sind Zubereitungen mit desodorierender Wirkung, enthaltend
a) das Zinksalz der Ricinolsäure,
b) aminofunktionelle Aminosäuren,
c) Lösungsvermittler,
d) organische und/oder anorganische Säuren und gegebenenfalls
e) Wasser.

## Beschreibung

Gegenstand der vorliegenden Erfindung sind Zubereitungen mit desodorierender Wirkung, enthaltend das Zinksalz der Ricinolsäure und mindestens eine aminofunktionelle Aminosäure, deren Salze und/oder Derivate.

Schweißbildung ist eine normale und gesunde Körperfunktion. Schweiß an sich ist geruchlos. Durch den Abbau der im Schweiß enthaltenden Eiweißverbindungen durch natürliche, grampositive Hautbakterien entsteht allerdings Körpergeruch, welcher in der modernen Gesellschaft als aufdringlich, abstoßend und ungepflegt empfunden wird. Für die Körperpflege wurden deshalb eine Vielzahl kosmetischer Produkte entwickelt, mit dem Ziel diesen unangenehmen Schweißgeruch zu beseitigen. Darreichungsformen solcher Produkte, welche sowohl geruchshemmende Deodorantien wie auch schweißhemmende Antitranspirantien einschließen, sind Stifte und Cremes, Seifen, Roll-ons wie auch Aerosol- und Pumpsprays.

Es gibt verschiedene Wirkprinzipien, welche in diesen handelsüblichen kosmetischen Rezepturen zum Tragen kommen. Deodorants überdecken die unangenehmen Gerüche durch Zusatz von Parfümstoffen. In der Regel sind in Deodorants auch antimikrobielle Wirkstoffe wie Triclosan®, ätherische Öle oder Farnesol® enthalten. Solche Materialien wirken als Bakterizide oder Bakteriostatika und reduzieren die natürliche Bakterienflora auf der Haut, wodurch die Geruchsbildung verhindert wird. Eine weitere Möglichkeit ist die Verwendung von Antitranspirantien oder Antiperspirantien, welche die Schweißabsonderung reduzieren. Als Wirkstoffe werden Aluminium- und Zirkoniumsalze verwendet, welche aufgrund ihrer eiweißfällenden Natur die Schweißdrüsen verengen und so die Schweißbildung herabsetzen. Ebenfalls verwendet werden in Deo-Produkten auch Enzymblocker wie Triethylcitrat, welche in den enzymatischen Mechanismus der bakteriellen Schweißzersetzung eingreifen, indem sie durch Inaktivierung der esterspaltenden Lipasen der Entstehung unangenehm riechender Abbauprodukte vorbeugen.

Des Weiteren sind auch so genannte Geruchsabsorber bekannt, bei denen es sich um Stoffe handelt, welche die geruchsbildenden Verbindungen durch Adsorption bzw. Absorption chemisch bzw. physikalisch binden können. Ein Vertreter derartiger Materialien sind Zinksalze der Ricinolsäure, deren Herstellung in der DE-B-17 92 074 beschrieben ist.

In der Patentliteratur finden sich auch Beschreibungen in denen diese Zinksalze in Kombinationen mit Zinksalzen der Abietinsäure oder mit Zinksalzen anderer gesättigter oder ungesättigter hydroxylierter Fettsäuren mit 16 und mehr C-Atomen sowie anderen oben angeführten Wirkstoffen eingesetzt werden.

Zinkricinoleat kann geruchsintensive organische Substanzen mit schwefel- bzw. stickstoffhaltigen funktionellen Gruppen, wie zum Beispiel Mercaptane, Thioether, niedermolekulare Carbonsäuren wie Isovalerinsäure oder auch Amine chemisch binden. Ein besonderer Vorteil dieser Art der Geruchslöschung ist, dass das bakterielle Gleichgewicht der Hautflora hierdurch nicht negativ beeinträchtigt wird.

Die Fähigkeit des Zinkricinoleats, Substanzen dieser Art chemisch fest zu binden, ermöglicht darüber hinaus auch den Einsatz in industriellen Anwendungsbereichen zum Reduzieren von unangenehmen Haushalts- und Industriegerüchen.

Zinkricinoleat ist allerdings aufgrund seiner polymeren Salzstruktur nur bedingt direkt einsetzbar. Es handelt sich um eine, in gängigen kosmetischen Lösungsmitteln, nur schwer lösliche Verbindung. Um wirksame Zubereitungen zu erhalten, muss es daher in Kombination mit Lösungsmitteln und Lösungsvermittlern eingesetzt werden. Als Lösungsmittel werden meistens ein- oder mehrwertige Alkohole, gegebenenfalls unter Zusatz von Wasser, verwendet. Üblicherweise eingesetzte hochethoxylierte Lösungsvermittler vermögen auch in hohen Konzentrationen das Zinkricinoleat allein nicht in Lösung zu halten und es werden dadurch auch keine fließfähigen Produkte erhalten.

Beispiele für spezielle Lösungsvermittler für Zinkricinoleat lassen sich der Patentliteratur entnehmen. So werden in der Patentschrift DE-B-37 26 636 Desodorantien auf Basis von Zinkricinoleat mit Lösungsmitteln und Lösungsvermittler beschrieben, wobei als Lösungsvermittler die hydrolysierten En-Addukte von Ricenfettsäuren und Maleinsäureanhydrid verwendet werden.

In der Patentschrift DE-B-38 08 114 werden ebenfalls Desodorantien auf Basis von Zinkricinoleat mit Lösungsmittel und Lösungsvermittlern beschrieben. Als Lösungsvermittler werden Partialester von Di- oder Polyhydroxyalkanen, Mono- und Disacchariden, Polyethylenglykolen oder Alkanolaminen mit den En-Addukten von Maleinsäureanhydrid an mindestens einfach ungesättigten Carbonsäuren mit einer Kettenlänge von 10 bis 25 Kohlenstoffatomen und Säurezahlen von 10 bis 140 beschrieben, welche vorzugsweise mit Amino- und/oder Amidoverbindungen wie Triethanolamin oder Glykolester der Asparagin- und der Glutaminsäure durch Bildung salzartiger Bindungen auf pH-Werte um 6,5 gepuffert werden.

Zubereitungen mit diesen Lösungsvermittlern waren jedoch nicht fließfähig und die daraus formulierten Deodorantlösungen neigen bereits bei sehr geringen Wassergehalten zur Trübung und Ausfällung einzelner Komponenten (vergl. DE-A-40 14 055 Seite 2, Zeilen 50-52).

Eine Verbesserung stellt die in der Patentschrift DE-B-40 14 055 vorgeschlagene Lösung dar, in welcher die Anmelder Mittel mit desodorierender Wirkung beschreiben, enthaltend das Zinksalz der Ricinolsäure und/oder das Zinksalz der Abietinsäure und/oder weitere Zinksalze anderer gesättigter oder ungesättigter hydroxylierter Fettsäuren mit 16 und mehr C-Atomen sowie 5 bis 50 Gew.-% eines ethoxylierten Fettalkohols mit gerader oder verzweigter Alkylkette und einer Kohlenstoffzahl zwischen 10 und 18 mit weniger als 30 Ethylenoxideinheiten pro Molekül und 5 bis 30 Gew.-% eines tertiären Amins.

Als tertiäre Amine werden tertiäre Aminoalkohole beansprucht, wie etwa Triethanolamin oder N,N,N',N'-Tetrakis(2-hydroxypropyl)ethylendiamin.

Die enthaltenen Aminoalkohole stellen für technische Anwendungen, in denen kein Hautkontakt gegeben ist, kein Problem dar. Für den Einsatz in Anwendungen mit Hautkontakt, wie insbesondere in Kosmetikprodukten, sind dagegen mehr und mehr Alternativen zu Aminoalkoholen gefragt, da diese oft Verunreinigungen von sekundären Aminen enthalten können. Außerdem sind diese Substanzen, auch wegen bekannt gewordener sensibilisierender und allergischer Reaktionen bei ihrer Anwendung auf der Haut, negativ in die Schlagzeilen gerückt.

Obwohl diese Formulierungen bereits einen recht guten Fortschritt darstellen, gibt es jedoch noch weiteren Verbesserungsbedarf. So ist auch hier die Wasserlöslichkeit bzw. Wasserverdünnbarkeit der Formulierungen ohne zusätzliche einund mehrwertige Alkohole als Lösungsvermittler noch immer nicht zufriedenstellend gelöst.

Es besteht daher weiterhin ein Bedarf an verbesserten Formulierungen für Zinkricinoleat, welche nicht nur gegenüber dem Stand der Technik eine erheblich verbesserte Wasserlöslichkeit aufweisen und in der Lage sind, die Löslichkeit des Zinkricinoleat in dem desodorierenden Mittel zu verbessern und auch bei längerer Lagerzeit und hohem Wassergehalt stabil in Lösung zu halten, sondern zusätzlich auch auf Rohstoffen basieren, welche in der Kosmetik wegen ihrer unbedenklichen und hautfreundlichen Eigenschaften allgemein akzeptiert sind und möglichst auch einen deutlichen Zusatznutzen aufweisen.

Ein solcher Zusatznutzen ist ein positiver Faktor, da Verbraucher erwarten, dass Desodorantien wie andere kosmetische Produkte zur Körperpflege auch, multiple bzw. multifunktionelle Eigenschaften aufweisen sollen. Neben der Deodorantwirkung können z.B. die positive Beeinflussung der Nährstoffversorgung oder auch der Regeneration der Haut von speziellem Interesse sein.

Neben Mildheit und Hautverträglichkeit ist auch eine vermehrte Nachfrage nach alkoholfreien Produkten zu verzeichnen, da alkoholhaltige Formulierungen vielfach ebenfalls zu Hautreizungen führen können.

Auch für den Anwendungsbereich Haushalts- und Industriegerüche sind diese Zusatznutzen von Interesse, da es sich hier hauptsächlich um wässrige Systeme handelt, die teilweise auch noch verdünnt werden. Durch die Auswahl der verwendeten Hilfsstoffe kann gleichzeitig eine bessere ökologische Verträglichkeit des desodorierenden Systems erzielt werden.

Vorzugsweise sollen Formulierungen mit Zinkricinoleat daher solche Rohstoffe als Lösungsvermittler enthalten, die nicht toxisch sind, vorzugsweise natürlichen Ursprungs sind, sehr gut von der Haut toleriert werden, eine hohe Verträglichkeit mit anderen Inhaltsstoffen aufweisen und sich problemlos in Deodorantien einarbeiten lassen. Besonders wünschenswert ist es, wenn diese Rohstoffe zusätzlich auch die Funktion bestimmter Komponenten ausüben kann, die bisher in Deodorantanwendungen eingesetzt werden, wie etwa pH-Regulatoren, Mittel zur Hautberuhigung oder Entzündungshemmung.

Es ist eine Aufgabe der Erfindung, solche Formulierungen mit desodorierender Wirkung zur Verfügung zu stellen.

Überraschenderweise wurde gefunden, dass durch die Mitverwendung von aminofunktionellen Aminosäuren deren Salze und/oder Derivate Mittel mit desodorierender Wirkung erhalten werden, welche alle diese gewünschten Kriterien erfüllen.

Ein Gegenstand der Erfindung sind daher Zubereitungen mit desodorierender Wirkung, enthaltend
a) Zinksalz der Ricinolsäure,
b) aminofunktionelle Aminosäuren,
c) Lösungsvermittler,
d) organische und/oder anorganische Säuren und gegebenenfalls
e) Wasser.

Weitere Gegenstände der Erfindung sind Verwendungen dieser Zubereitungen zur Herstellung von desodorierend wirkenden hygienischen und kosmetischen Formulierungen, desodorierend wirkenden Haushaltsreinigern, Industriereinigern, Adsorbern in Filtern, desodorierend wirkenden Formulierungen für die Anwendung in der privaten und gewerblichen Tierhaltung, desodorierend wirkenden Formulierungen für die Behandlung von textilen Fasern und Geweben.

Die erfindungsgemäß mitverwendbaren Zinksalze der Ricinolsäure sind die im Handel unter den jeweiligen Markennamen erhältlichen Produkte wie insbesondere das TEGO® Sorb, TEGODEO® oder ORTEGOL® 2000 der Firma Goldschmidt AG.

Das Zinksalz der Ricinolsäure kann in Mengen von 0,01 bis 60 Gew.-%, vorzugsweise in Mengen von 20 bis 60 Gew.-%, bezogen auf das Wirkstoffkonzentrat, mitverwendet werden.

Als aminofunktionelle Aminosäuren werden erfindungsgemäß bevorzugt Lysin, Hydroxylysin und Arginin sowie deren Derivate und Salze mit organischen oder anorganischen Säuren allein oder in Mischung miteinander und untereinander mitverwendet. Geringe Anteile weiterer natürlicher oder synthetischer Aminosäuren sind ebenfalls möglich.

Die Verwendung von Aminosäuren als Wirkstoffe in kosmetischen Anwendungen ist bereits vielfach beschrieben. Aufgrund ihrer zwitterionischen Natur wirken Aminosäuren als Puffer und stabilisieren den Säureschutzmantel der Haut.

L-Arginin ist eine körpereigene, semiessentielle Aminosäure welche in Mangelsituationen durch die Nahrung ergänzt werden muss. Es wirkt hautbefeuchtend und gehört zu den natürlichen Feuchthaltefaktoren der Haut (NMF). Ein Arginin-Mangel ist mit einem verminderten Harnstoffgehalt der Haut ursächlich verknüpft, da Harnstoff ein metabolisches Folgeprodukt des Arginins ist. Dadurch wird der natürliche Feuchthaltegradient der Haut beeinträchtigt und es kann zu Phänomenen wie trockener Haut, Riss- und Schuppenbildung kommen. Einen Überblick über Erfahrungen mit topisch appliziertem L-Arginin findet sich bei Wohlrab J, Marsch W Ch. in Z. Hautkr. 2000; 75: 485-486.

Lysin ist ein Teil vieler Proteine und hat entsprechend viele Funktionen im Körper. Es trägt zum Wachstum, zur Gewebereparatur, zur Bildung von Enzymen, Hormonen und Antikörpern sowie zur Kollagensynthese und Knochengesundheit bei. Lysin hilft möglicherweise, Kalzium zu absorbieren und zu speichern. Lysin hält weiter die Stickstoffbalance im Körper aufrecht und es spielt eine wichtige Rolle im Immunsystem. Bekannt sind beispielsweise antivirale Fähigkeiten.

Sowohl durch Lysin wie auch Arginin kann die Haut- und Schleimhautverträglichkeit von reizenden Substanzen außerordentlich verbessert werden.

Gemäß einer weiteren Ausführungsform können die erfindungsgemäßen Zubereitungen auch Derivate der aminofunktionellen Aminosäuren enthalten. Dazu zählen Arginin- und Lysinsalze, insbesondere solche mit langkettigen Fettsäuren, welche aufgrund ihrer oberflächenaktiven Eigenschaften als milde, hautfreundliche Tenside wirken. Derartige Materialien werden z.B. unter dem Handelsnamen "Aminosoap" von der Firma Ajinomoto kommerziell angeboten. Ebenfalls eingesetzt werden können N-Acylderivate des Lysins und Arginins und kationisch modifizierte fettchemisch modifizierte Varianten hiervon.

Die aminofunktionellen Aminosäuren können in Mengen von 0,01 bis 40 Gew.-%, vorzugsweise in Mengen von 0,1 bis 30 Gew.-%, bezogen auf die Gesamtformulierung, mitverwendet werden.

Lösungsvermittler im Sinne der vorliegenden Erfindung sind nichtionische und ionische Tenside wie Alkoxilate, Polyglycerine, Glykolether, Glykole, Polyethylenglykole, Polypropylenglykole, Polybutylenglykole, Glycerinesterethoxylate, Polysorbate, Alkylethersulfaten, Alkyl- und/oder Arylsulfonaten, Alkylsulfaten, Estersulfonate (Sulfofettsäureester), Ligninsulfonate, Fettsäurecyanamide, anionische Sulfobernsteinsäuretenside, Fettsäureisethionate, Acylaminoalkansulfonate (Fettsäuretauride), Fettsäuresarcosinate, Ethercarbonsäuren und Alkyl(ether)-Phosphate.

Bevorzugte nichtionische Lösungsvermittler sind die, gegebenenfalls einseitig mit einem C₁₋C₆-Alkanol veretherten, C₂₋C₆-Alkylenglykole und Poly-C₂₋C₃-alkylenglykolether mit durchschnittlich 1 bis 9 gleichen oder verschiedenen, vorzugsweise gleichen, Alkylenglykolgruppen pro Molekül wie auch Alkohole und Fettalkoholpolyglykolether, vorzugsweise Propylenglykol, Dipropylenglykol, Trimethylolpropan sowie niederethoxyilierte Fettalkohole mit 6 bis 22, vorzugsweise 8 bis 18, insbesondere 8 bis 12 und besonders bevorzugt 8 bis 11 Kohlenstoffatomen.

Bevorzugte ionische Lösungsvermittler sind Alkylethersulfate, Sulfobernsteinsäuretenside, Polyacrylate und Phosphonsäuren, vorzugsweise Laurylsulfat, Laurylethersulfat, Natriumsulfobernsteinsäurediisooctylester, 1-Hydroxyethan-1,1-Diphosphonsäure sowie Diacetylweinsäureester.

Sie können in Mengen von 0,01 bis 40 Gew.-%, vorzugsweise in Mengen von 0,1 bis 30 Gew.-%, bezogen auf die Gesamtformulierung, mitverwendet werden.

Als organische und/oder anorganische Säuren werden insbesondere HCl, Phosphorsäure, Essigsäure, Milchsäure und/oder Citronensäure eingesetzt. Die verwendete Menge ist im wesentlichen abhängig von der Art und der Menge der mitverwendeten Aminosäure und sollte in jedem Falle zur Einstellung eines pH-Wertes der Gesamtformulierung von ca. 3 bis 9 genügen. Mengen von 0,01 bis 10 Gew.-% sind hierzu in der Regel ausreichend.

Als Hilfs- und Zusatzstoffe werden die auf dem jeweiligen Einsatzgebiet bekannten, überwiegend CTFA gelisteten, Produkte in den üblichen Mengen eingesetzt.

Als Wasser kann entsalztes oder übliches Leitungswasser, vorzugsweise solches mit nicht zu hohen Härtegraden, eingesetzt werden.

Die erfindungsgemäßen Mittel können hergestellt werden durch Mischen der Komponenten in üblicher Weise wie beispielsweise durch gleichzeitige, portionsweise oder aufeinander folgende Zugabe von Aminosäure und organischer Säure zu dem vorgelegten, intensiv gerührten und gegebenenfalls erwärmten Lösungsvermittler und anschließender Zudosierung der weiteren gegebenenfalls mitverwendeten Komponenten.

Die erfindungsgemäßen Wirkstoffkombinationen können ebenso wie die im Markt befindlichen, bekannten Zinkricinoleate in alle üblichen Deoformulierungen eingearbeitet werden. Dazu zählen Pump-Spraylösungen, Flüssigseifen, Deo-Sprays und Deo-Cremes. Ebenso ist es möglich die entsprechenden Formulierungen auch anderen üblicherweise verwendeten kosmetischen Produkten wie z.B. Haarshampoos oder Haarspülungen zuzusetzen, sofern bekannte Inkompatibilitäten dies nicht von vornherein ausschließen.

Auch können diese Wirkstoffkombinationen in Reinigungsmittelformulierungen Verwendung finden wie z.B. in Handgeschirrspülmitteln, Allzweckreinigern, Teppichreinigern, Tränktüchern (deowipes) oder als Raumspray, desodorierend wirkenden Haushaltsreinigern, Industriereinigern, Adsorbern in Filtern, desodorierend wirkenden Formulierungen für die Anwendung in der privaten und gewerblichen Tierhaltung, desodorierend wirkenden Formulierungen für die Behandlung von textilen Fasern und Geweben.

Die zusätzliche Verwendung von Alkoholen, insbesondere Ethanol, oder Isopropylalkohol, ist möglich und soll nicht a priori ausgeschlossen werden, da solche Zusätze unter ästhetischen oder auch konservierenden Aspekten dort mitverwendet werden können, wo keine Hautirritationen befürchtet werden müssen.

Es war jedoch völlig überraschend, dass mit den im Rahmen dieser Erfindung beanspruchten Wirkstoffkombinationen auch stabile, alkoholfreie Deodorantformulierungen hergestellt werden können, welche in verdünnten, wässrigen Lösungen nicht ausfallen und dadurch inaktiviert werden.

Die erfindungsgemäßen Desodorantien, ihre Herstellung und Anwendung sind in den nachfolgenden Beispielen näher erläutert. Die chemische Charakterisierung der in den Beispielen genannten Handelsnamen ist Tabelle 1 zu entnehmen.

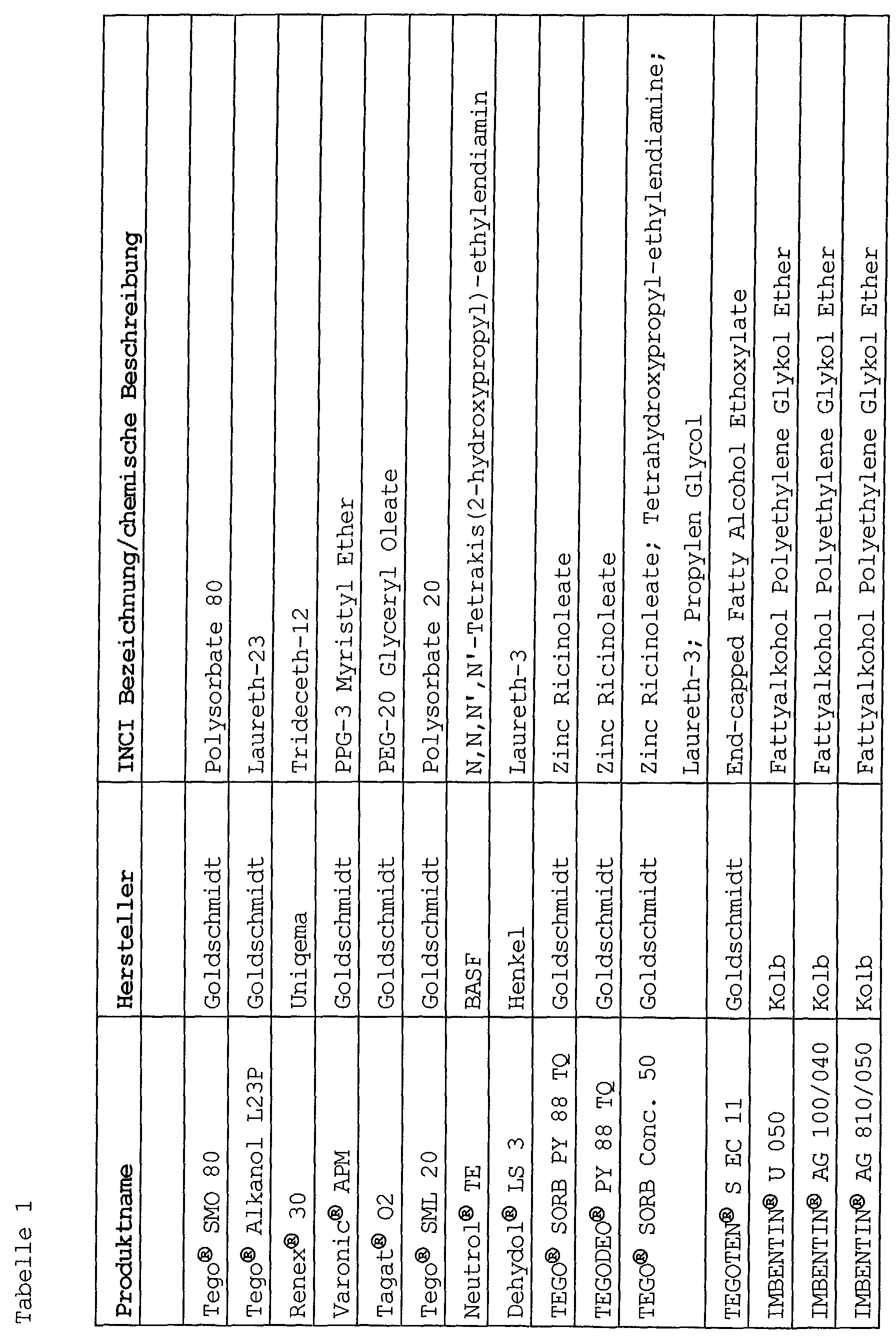

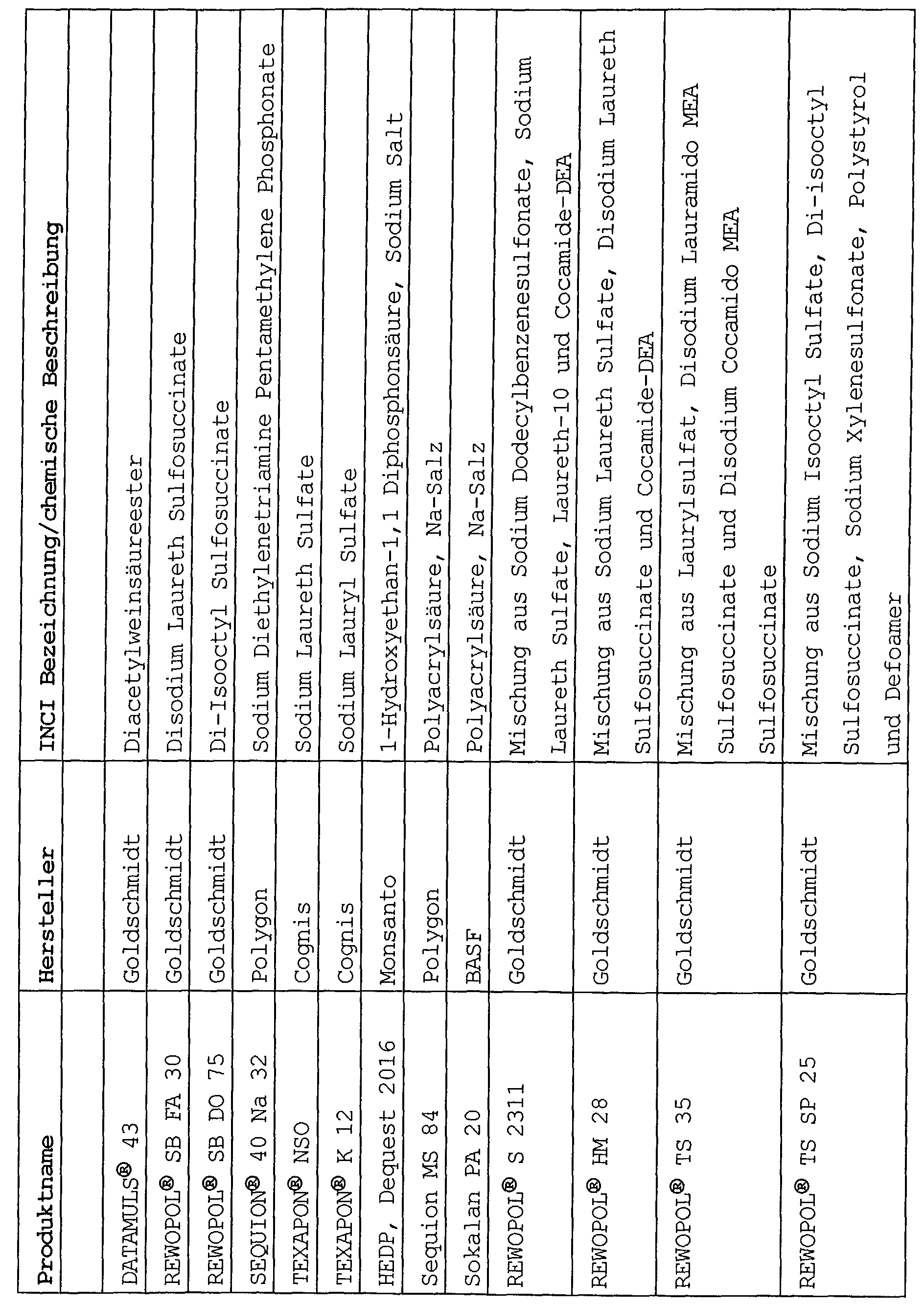

In den nachfolgenden Beispielen werden bevorzugte Zusammensetzungen der erfindungsgemäßen Wirkstoffkonzentrate beschrieben.

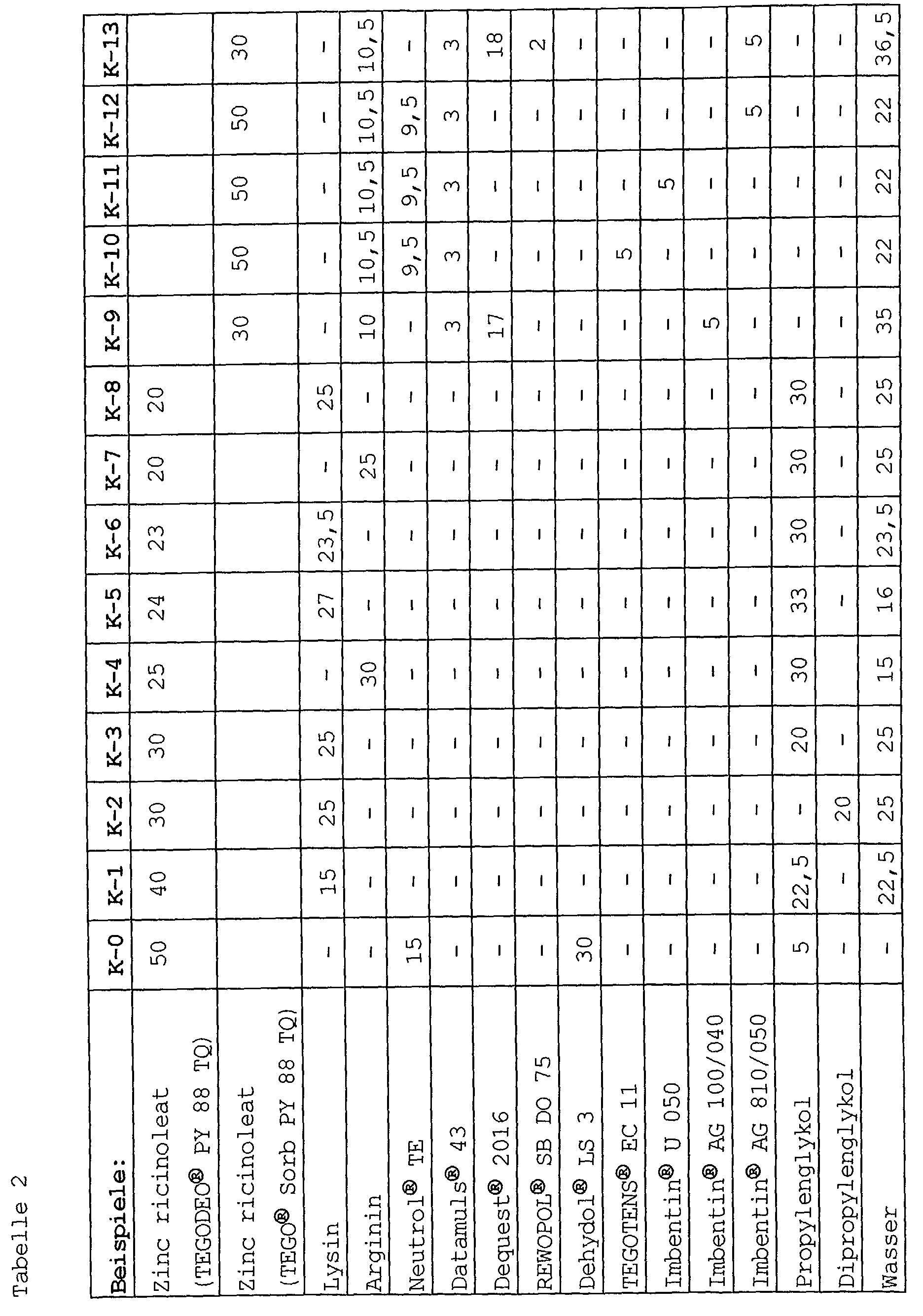

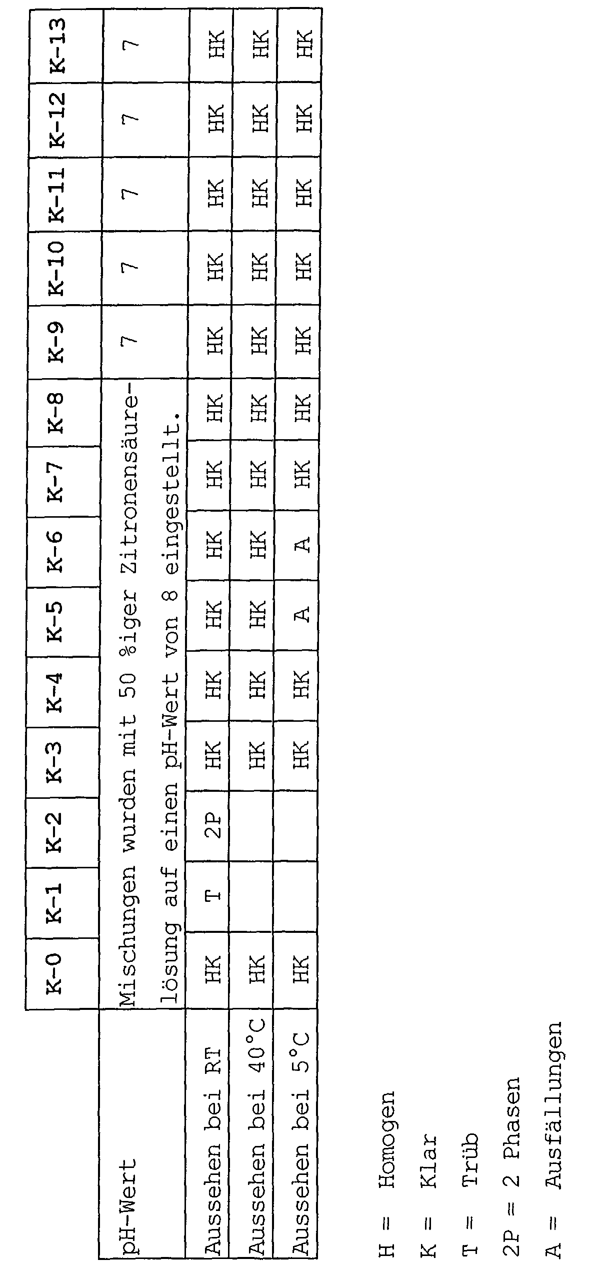

Der Nachweis der besseren Wasserverträglichkeit der erfindungsgemäßen Mittel mit desodorierender Wirkung soll anhand der folgenden Beispiele von Pump-Sprayrezepturen dargestellt werden.

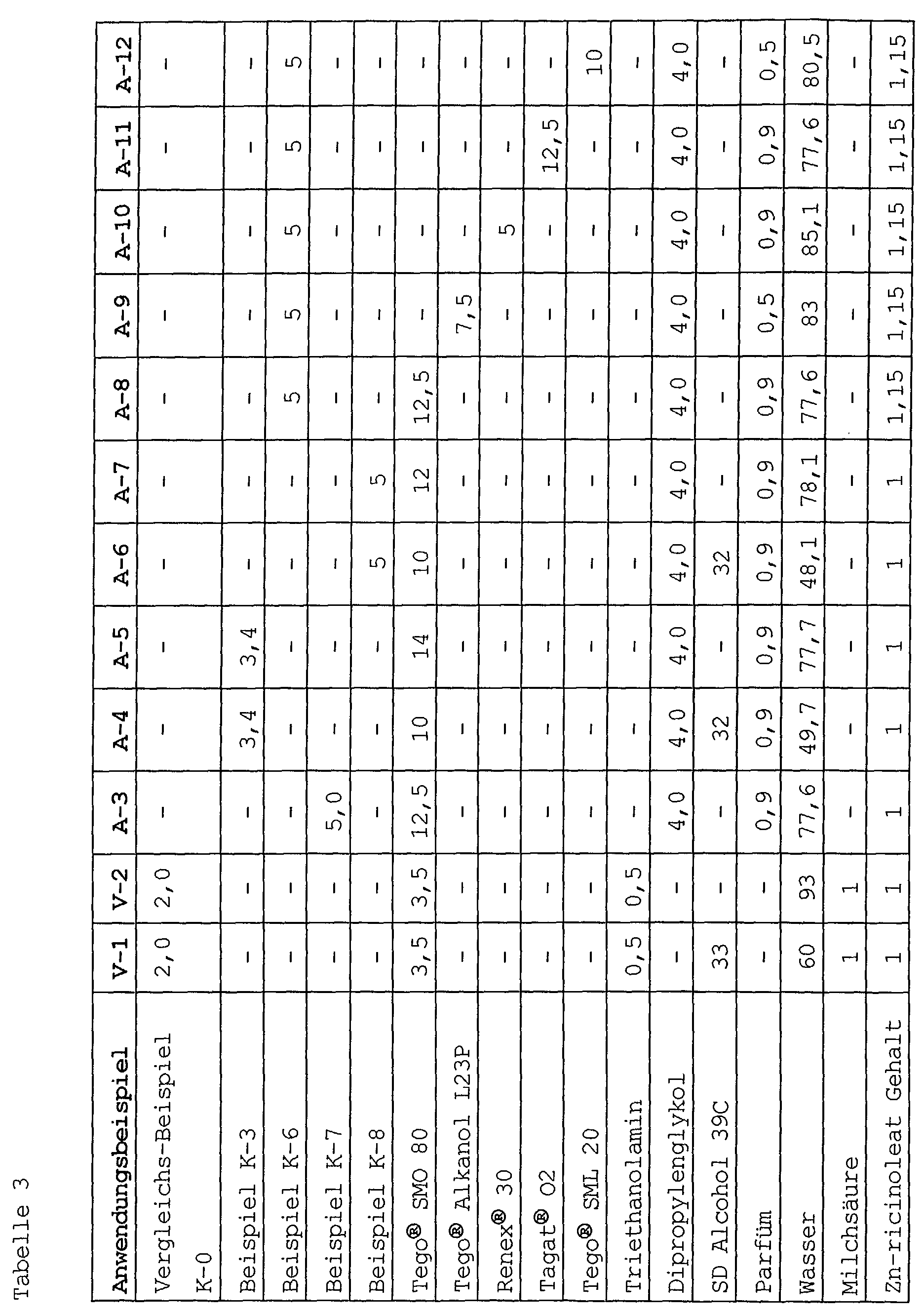

Alle Pump-Spraylösungen wurden mit 50 %iger Zitronensäurelösung auf einen pH-Wert von 5,5 eingestellt. In allen erfindungsgemäßen Fällen wurden nach dem Mischen klare Lösungen erhalten, die selbst nach 3 Monaten Lagerung bei Temperaturen von 40 °C sowie 10 Tau-Gefrier-Zyklen noch klar blieben. Die mit Vergleichsbeispiel K-0 hergestellten Lösungen werden bei Raumtemperatur trüb und es kommt zu Ausfällungen.

**Tabelle 4**

| Mit Beispiel K-6 wurde ein Deostick entwickelt. Stick-Formulierung | |
|---|---|
| | **Beispiel A-13** |
| Varonic® APM | 70,5 |
| Na-Stearat | 8,0 |
| Propylenglykol | 10,0 |
| Wasser | 3,0 |
| Parfüm | 1,0 |
| Beispiel K-6 | 7,5 |
| Zn-ricinoleat Gehalt | 1,7 |

Diese Formulierung ist nach 3 Monaten bei 40 °C und 10 Tau-Gefrier-Zyklen noch stabil.

Für eine Verwendung in Reinigungslösungen spielen die bessere Wasserverträglichkeit und die reduzierte Rückstandsbildung auf harten Oberflächen eine wesentliche Rolle. Mit Beispielen von nicht verdünnbaren Reinigern im Vergleich zu den erfindungsgemäßen Beispielen die sich durch einen höheren Wirkstoffgehalt auszeichnen sollen die Unterschiede herausgestellt werden.

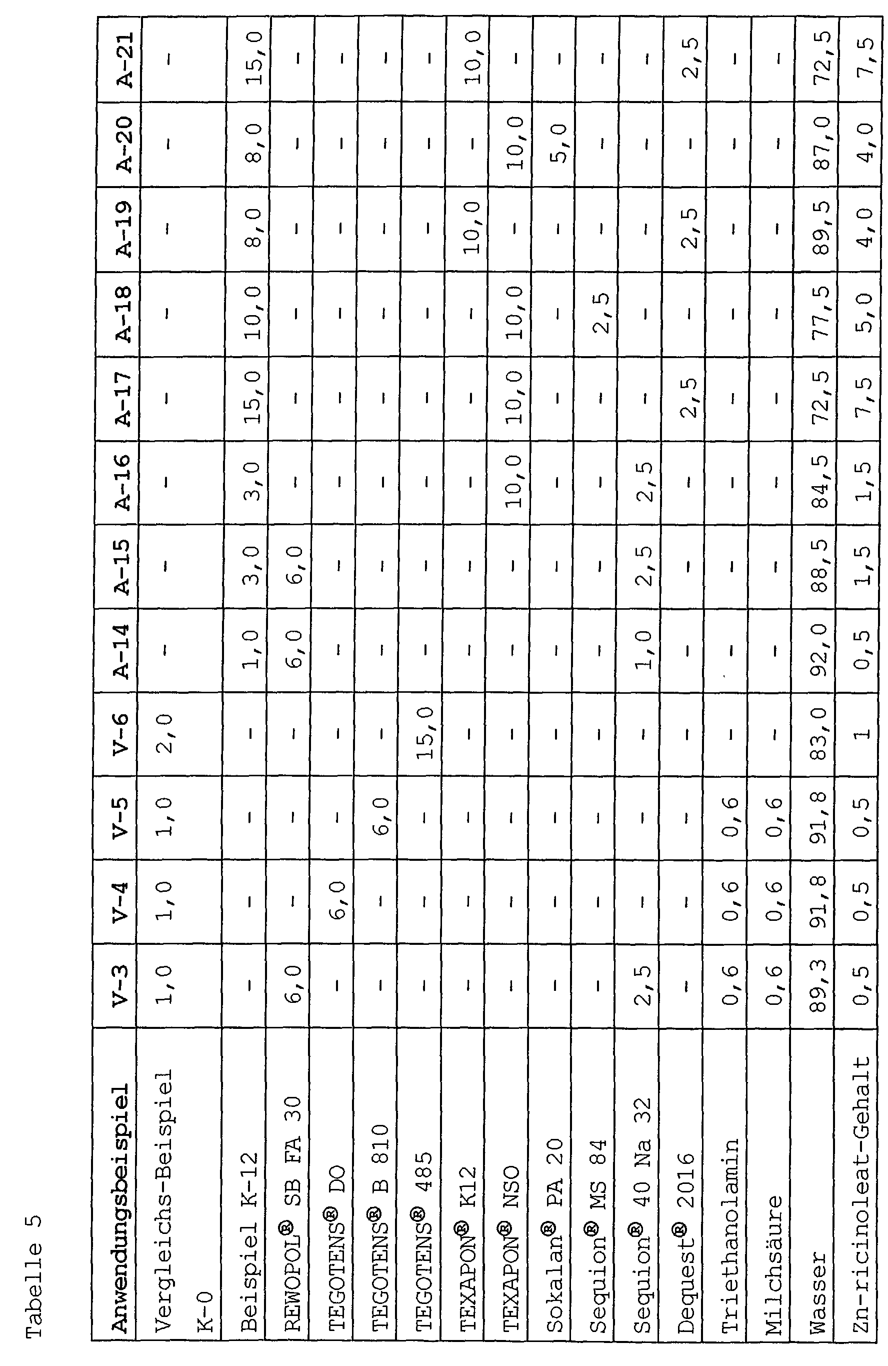

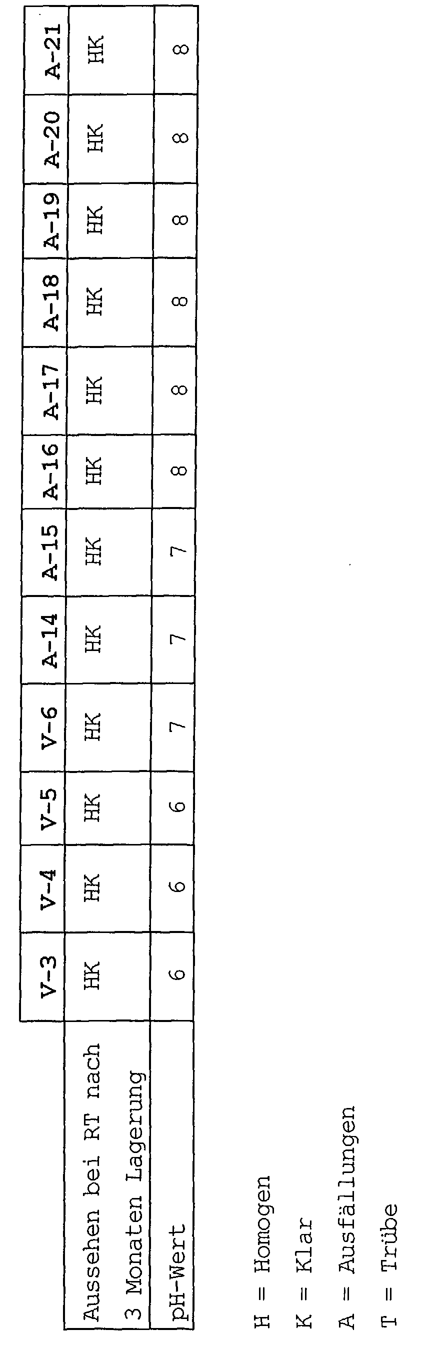

Die Verarbeitung in Reinigungsmittelkonzentraten wurde geprüft, um die einfachere Einarbeitung in bekannten Tensidsystemen zu dokumentieren. Die verwendeten Tensidsysteme sind Konzentrate zur einfachen Herstellung der jeweiligen Anwendungsfelder.

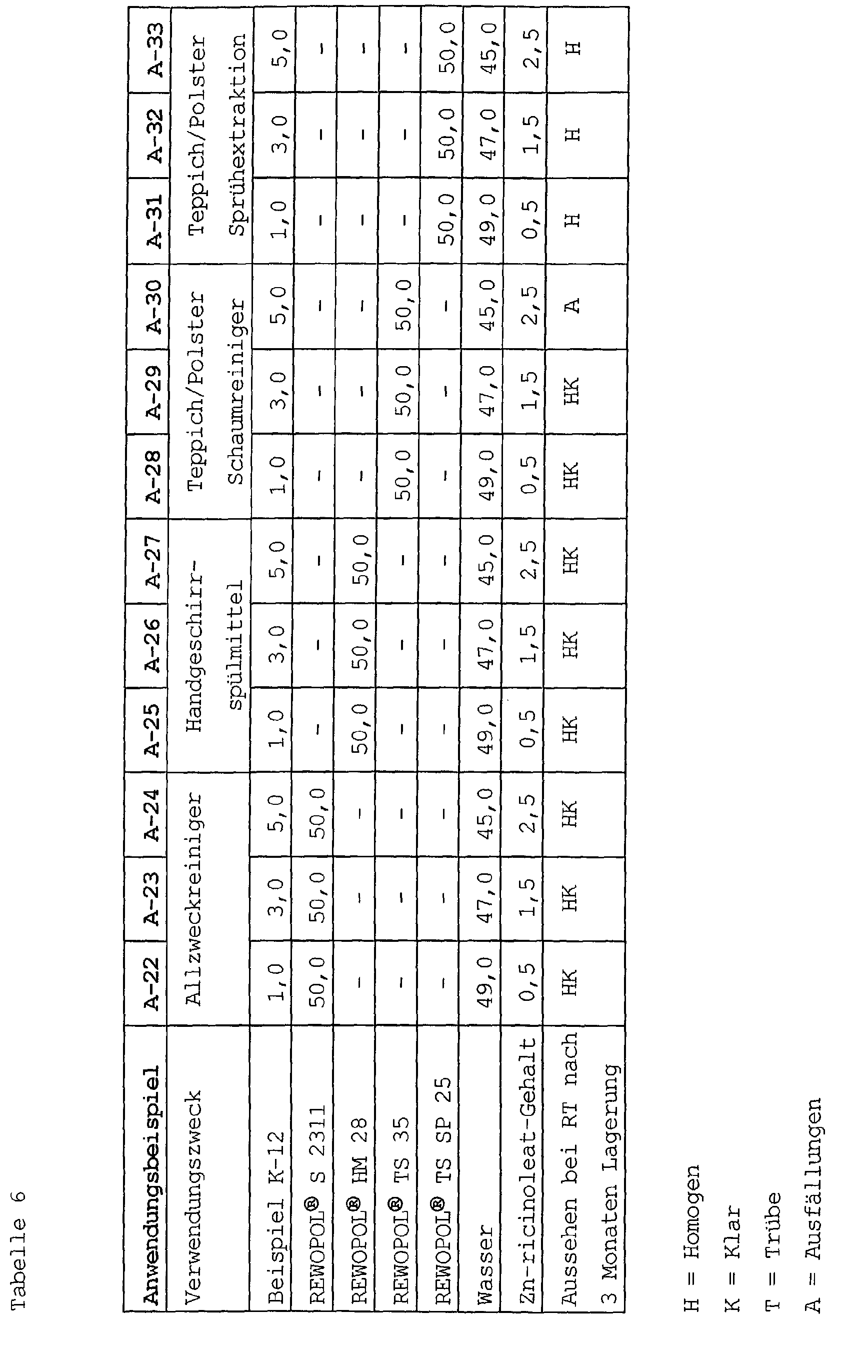

## Patentansprüche

1. Zubereitungen mit desodorierender Wirkung, **dadurch gekennzeichnet, dass** sie enthalten
a) das Zinksalz der Ricinolsäure,
b) aminofunktionelle Aminosäuren,
c) Lösungsvermittler,
d) organische und/oder anorganische Säuren und gegebenenfalls
e) Wasser.

2. Zubereitungen mit desodorierender Wirkung gemäß Anspruch 1, **dadurch gekennzeichnet, dass** sie enthalten
a) 0,01 bis 60 Gew.-% Zinksalz der Ricinolsäure,
b) 0,01 bis 40 Gew.-% aminofunktionelle Aminosäuren,
c) 0,01 bis 40 Gew.-% Lösungsvermittler,
d) 0,01 bis 10 Gew.-% organische und/oder anorganische Säuren und gegebenenfalls
e) ad 100 Gew.-% Wasser.

3. Zubereitungen mit desodorierender Wirkung gemäß Ansprüche 1 und 2, **dadurch gekennzeichnet, dass** sie als aminofunktionelle Aminosäuren mindestens eine der Verbindungen Lysin, Hydroxylysin, Arginin und/oder deren Derivate oder Salze enthalten.

4. Zubereitungen mit desodorierender Wirkung gemäß Ansprüche 1 bis 3, **dadurch gekennzeichnet, dass** sie als Lösungsvermittler ionische Tenside, nichtionische Tenside und/oder Glykole enthalten.

5. Zubereitungen mit desodorierender Wirkung gemäß Ansprüche 1 bis 4, **dadurch gekennzeichnet, dass** sie als organische Säuren Essigsäure, Milchsäure und/oder Citronensäure enthalten.

6. Verwendung der Zubereitungen gemäß Anspruch 1 zur Herstellung von desodorierend wirkenden hygienischen und kosmetischen Formulierungen.

7. Verwendung der Zubereitungen gemäß Anspruch 1 zur Herstellung von Deodorantien.

8. Verwendung der Zubereitungen gemäß Anspruch 1 zur Herstellung desodorierend wirkenden Haushaltsreinigern, Industriereinigern, Adsorbern in Filtern.

9. Verwendung der Zubereitungen gemäß Anspruch 1 zur Herstellung von desodorierend wirkenden Formulierungen für die Anwendung in der privaten und gewerblichen Tierhaltung.

10. Verwendung der Zubereitungen gemäß Anspruch 1 zur Herstellung desodorierend wirkenden Formulierungen für die Behandlung von textilen Fasern und Geweben.
